# FASCICULE DE BREVET EUROPEEN

(11) **EP 0 693 279 B1**
(45) Date de publication et mention de la délivrance du brevet: **16.10.1996**
(21) Numéro de dépôt: 95401189.6
(22) Date de dépôt: 22.05.1995
(51) Int. Cl.: A61K 7/155, A61K 7/00

(54) **Composition dépilatoire solide contenant un agent structurant particulaire**
Eine teilchenförmige strukturgebende Substanz enthaltendes festes Enthaarungsmittel
A solid depilatory composition containing a particulated structurant

(30) Priorité: 11.07.1994 FR 9408564
(43) Date de publication de la demande: 24.01.1996
(73) Titulaire: L'OREAL, F-75008 Paris (FR)
(72) Inventeur: De La Mettrie, Roland, F-78110 Le Vesinet (FR); De Labbey, Arnaud, F-93600 Aulnay Sous Bois (FR); N'Guyen, Lylan, F-92240 L'Hay Les Roses (FR)
(74) Mandataire: Lhoste, Catherine

(56) Documents cités:
- EP-A- 0 287 773
- WO-A-89/00041
- WO-A-94/14402
- DE-A- 965 920
- FR-A- 2 631 825
- DATABASE WPI Week 8246 Derwent Publications Ltd., London, GB; AN 82-98267E & JP-A-57 163 308 (SHINMEIWA IND KK) , 7 Octobre 1982

## Description

La présente invention a pour objet une nouvelle composition dépilatoire ayant un aspect de solide déformable. Du fait de son extrème douceur, cette composition peut être appliquée aussi bien sur le visage que sur le corps, et notamment autour des lèvres, sur les bras, sur les jambes et sous les aisselles.

L'invention se rapporte aussi à un procédé de traitement cosmétique pour l'élimination des poils superflus.

L'invention se rapporte encore à l'utilisation d'un agent structurant particulier dans une composition dépilatoire.

En dehors des moyens d'épilation mécanique tels que les rasoirs, les méthodes dépilatoires actuellement utilisées consistent à appliquer sur les parties à épiler des compositions dépilatoires telles que les cires ou les crèmes dépilatoires.

On applique les cires, le plus souvent après les avoir fait fondre, puis on les enlève après un certain temps de pose, ce qui entraîne ainsi l'arrachement des poils. L'épilation à la cire est malaisée quand il faut manipuler de la cire chaude, et elle est également douloureuse du fait que les poils sont arrachés violemment quand on enlève la cire durcie. De plus, l'épilation obtenue n'est pas toujours satisfaisante.

De façon simplifiée, une crème dépilatoire comprend un agent ramollissant le poil, le plus souvent un thiol, dans un support ou un milieu cosmétiquement acceptable de pH élevé, par exemple de l'ordre de 12 ou 12,5. La crème dépilatoire est appliquée à froid et fait tomber les poils en les ramollissant. Les crèmes dépilatoires sont difficiles à doser en raison de leur fluidité et donnent souvent une épilation incomplète. De plus, après chaque traitement d'épilation de la peau, il est nécessaire d'éliminer la crème dépilatoire et de rincer la zone de peau épilée et, malheureusement, beaucoup de crèmes dépilatoires présentent l'inconvénient d'être difficiles à éliminer et/ou de laisser des traces de produits sur la peau.

En outre, quelle que soit la technique d'épilation choisie, elle est le plus souvent irritante pour la peau.

Par ailleurs, les utilisateurs recherchent, de plus en plus, de nouvelles textures et de nouveaux concepts de produits.
Il est connu du document DE-A-965920 des compositions contenant un agent de ramollissement des poils et des agents insolubles dans ces compositions.

La présente invention a justement pour objet une nouvelle composition dépilatoire permettant notamment de remédier aux inconvénients mentionnés ci-dessus. En particulier cette composition se rince de façon remarquable et présente une texture tout à fait inhabituelle. En outre, elle est simple à appliquer.
De façon surprenante, la demanderesse a trouvé qu'il était possible de conférer à une composition dépilatoire un aspect de solide déformable en utilisant un agent structurant ou texturant original.

Ainsi, l'invention se rapporte à une composition dépilatoire, caractérisée en ce qu'elle contient, dans un milieu cosmétiquement acceptable, un agent de ramollissement des poils et un agent structurant insoluble dans ce milieu et formé de particules solides, conférant à la composition un aspect de solide déformable dans lequel le milieu est emprisonné, cet agent étant apte à s'éliminer de la peau au moyen d'un diluant.

L'invention a aussi pour objet l'utilisation d'un agent structurant dans une composition dépilatoire pour lui conférer un aspect de solide déformable, cet agent étant formé de particules solides et étant apte à être éliminé au moyen d'un diluant.

L'un des avantages de cette texture solide est que la composition de l'invention ne risque pas de s'échapper de son conditionnement notamment lors de son transport. Par ailleurs cette composition est très facilement préhensible et ne s'écoute pas entre les doigts. Son dosage est beaucoup plus simple que celui des crèmes habituelles.

Selon l'invention, la composition se présente sous l'aspect d'un solide déformable, sec, ne tachant pas et ressemblant à de la guimauve (voir le document US-A-3 682 659 pour la consistance de la guimauve). Ce solide peut être modelé comme de la pâte à modeler pour enfants. Il peut être rompu facilement à la main afin de ne prélever que la quantité nécessaire de produit. En particulier, cette composition peut être conditionnée sous forme de monodose et par exemple sous forme de petits cubes, de billes ou de berlingots.

Grâce aux particules de l'invention, il est notamment possible d'obtenir une structure homogène (solide déformable) pour des constituants conduisant normalement à deux phases distinctes (constituants non miscibles par exemple huile/eau).

En vue d'obtenir un solide au toucher agréable et doux, il est préférable d'utiliser des particules ayant une granulométrie de 1 µm à 300 µm, par exemple de 5 µm à 200 µm et de préférence de 10 µm à 100 µm et mieux de 15 µm à 40 µm.

La grande douceur apportée par ces particules permet l'utilisation de la composition dépilatoire de l'invention pour les personnes à peau sensible.

Afin de conférer à la composition de l'invention, un aspect aéré et léger, on utilise avantageusement, des particules ayant une densité inférieure à 0,09 et mieux inférieure à 0,06 et encore mieux inférieure à 0,04.

En vue d'obtenir cette faible densité, on utilise avantageusement des particules creuses remplies d'un gaz. Ce gaz peut être de l'air, de l'azote, de l'isobutane, de l'isopentane, etc.
Selon une autre caractéristique avantageuse de l'invention, les particules se présentent sous forme de billes. Il est toutefois possible d'utiliser des particules ayant la forme de fibres.

Ces particules peuvent être réalisées en différents matériaux inertes ne réagissant pas chimiquement avec le milieu ou support cosmétiquement acceptable en particulier ces particules ne réagissent pas avec les huiles, les tensioactifs, l'eau et les différents autres constituants de la composition, tels que les actifs.

L'agent texturant de l'invention présente la particularité de s'éliminer facilement de la peau par simple dilution. Il joue en fait le rôle de véhicule ou de réservoir pour le support cosmétique. Il permet, en outre, de récupérer le support et notamment le ou les actifs, emprisonnés dans le solide déformable, lorsque c'est nécessaire par simple dilution à l'eau. Ceci est probablement dû au fait que le support cosmétique est logé dans les espaces interparticulaires du solide et non dans les particules.

Outre l'eau, on peut utiliser comme diluant de l'eau aditionnée d'un ou plusieurs solvants polaires comme les alcools inférieurs (éthanol, isopropanol) et les glycols (propylène glycol), d'un ou plusieurs tensioactifs. On peut aussi utiliser une eau chargée en sels.

Comme critère de choix de l'agent texturant, on peut réaliser le test suivant :
- ajout de particules déterminées dans de l'eau contenant un colorant classiquement utilisé dans le domaine dépilatoire tel que l'azulène, jusqu'à l'obtention d'une pâte colorée,
- versement d'une goutte d'eau sur la pâte.

Lorsque la pâte au point d'impact de la goutte d'eau est beaucoup plus claire que le reste de la pâte, cela signifie que les particules considérées sont candidates comme agent texturant. Inversement, lorsque la pâte au point d'impact ne s'est pas décolorée, les particules considérées ne sont nullement appropriées.

Les particules inertes sont avantageusement réalisées en verre ou en matériaux thermoplastiques comme les polyamides tels que le Nylon, les polymères ou copolymères d'acrylonitrile, de chlorure de vinylidène, de chlorure de vinyle et/ou de monomère acrylique ou styrénique, éventuellement expansés. Le monomère acrylique est par exemple un acrylate ou méthacrylate de méthyle ou d'éthyle. Le monomère styrénique est par exemple l'α-méthyl-styrène ou le styrène.

Comme particules de verre utilisables dans l'invention, on peut citer les billes de verres creuses vendues par la société 3M sous la référence Scotchlite Glass Bubbles S 22. 95 % de ces billes ont un diamètre inférieur à 74 µm.

Comme particules de Nylon, on peut utiliser les particules d"Orgasol" vendues par la société Atochem. Ces particules sont des sphères pleines, poreuses, de diamètre allant de 5 µm à 60 µm.

De préférence, les particules sont des particules creuses déformables d'un copolymère expansé de chlorure de vinylidène et d'acrylonitrile ou de chlorure de vinylidène, d'acrylonitrile et de méthacrylate. On peut par exemple utiliser un copolymère contenant : de 0 % à 60% de motifs dérivés du chlorure de vinylidène, de 20 % à 90% de motifs dérivés d'acrylonitrile et de 0 % à 50 % de motifs dérivés d'un monomère acrylique ou styrénique, la somme des pourcentages (en poids) étant égale à 100. Ces particules se présentent notamment à l'état sec ou hydraté et peuvent être obtenues, par exemple, selon les procédés décrits dans les brevets et demandes de brevet EP-A-56 219, EP-A-348 572, EP-A-320 473, EP-A-112 807 et US-A-3 615 972.

Ces particules creuses peuvent être par exemple celles formées d'un terpolymère d'acrylonitrile, de méthacrylate et de chlorure de vinylidène et vendues sous la marque EXPANCEL par la société Nobel Casco, et en particulier sous les références 551 DE 12 (granulométrie d'environ 12 µm et masse volumique 40 kg/m³), 551 DE 20 (granulométrie d'environ 30 µm et masse volumique 65 kg/m³), 551 DE 50 (granulométrie d'environ 40 µm).

On peut aussi utiliser des particules formées du même terpolymère et ayant une granulométrie d'environ 18 µm et une masse volumique d'environ 60 à 80 kg/m³, appelées ici EL 23, ou ayant une granulométrie d'environ 34 µm et une masse volumique d'environ 20 kg/m³, appelées ici EL 43, ou ayant une granulométrie d'environ 150 µm, appelées ici EL 55.

Comme autres particules creuses polymériques utilisables dans l'invention, on peut encore citer les polymères et les copolymères obtenus à partir des esters ou acides, itaconique, citraconique, maléique, fumarique, de l'acétate ou lactate de vinyle (voir à cet effet le document JP-A-2-112304), ou encore des particules de copolymère non expansé de chlorure de vinylidène et d'acrylonitrile ou de chlorure de vinylidène, d'acrylonitrile et de méthacrylate, vendues sous la marque EXPANCEL avec la référence 551 WU.

En revanche, les particules d'amidon de maïs, de silice pyrogénée, de polyéthylène, de polyuréthane ou de polyester non expansé ne permettent pas d'obtenir une composition solide qui s'élimine bien de la peau lors du rinçage.

Il est certes connu de modifier la viscosité d'un milieu liquide avec des particules solides (voir à ce sujet l'article Elsevier Sequoia, 1992, Progress in Organic Coatings, 21, p.255-267, de A. Toussaint "Choice of rheological model for steady flow : application to industrial concentrated suspensions") mais personne jusqu'à ce jour n'a ni décrit ni suggéré d'utiliser le produit solide, obtenu à partir d'une certaine concentration des particules, dans le domaine dépilatoire pour stocker le milieu dans lequel sont dispersées les particules.

Autrement dit, l'obtention ou non du solide déformable est liée à la quantité d'agent structurant utilisée dans la composition au-dessus d'une certaine quantité de particules, appelée charge pigmentaire volumique critique et notée CPVC, on note une augmentation brusque de la viscosité du milieu. La CPVC est fonction du milieu et de la nature des particules elle doit donc être déterminée à chaque fois. Sa détermination ne pose aucun problème pour l'homme du métier.

On peut, par exemple, utiliser la méthode officielle ASTM pour déterminer la CPVC.

L'invention a encore pour objet l'utilisation de la composition définie précédemment pour l'élimination des poils.

Ainsi, l'invention a encore pour objet un procédé d'épilation de la peau, consistant à appliquer sur la peau une composition telle définie ci-dessus, puis à rincer la peau.

La composition de l'invention contient, outre les particules texturantes, tous les constituants classiquement utilisés dans les compositions dépilatoires. Ces constituants sont notamment des agents de ramollissement du poil tels que les thiols et notamment le thioglycolate de calcium, des huiles minérales, végétales, synthétiques ou siliconées, de l'eau, des solvants alcooliques, des filtres, des parfums, des tensioactifs, des polymères, des conservateurs, des antioxydants, des agents régulateurs de pH, des séquestrants, des charges etc.

L'exemple ci-après est donné à titre illustratif et non limitatif en vue de mieux faire ressortir les caractéristiques de l'invention.

### EXEMPLE : PATE DEPILATOIRE

| | |
|---|---|
| - Thioglycolate de calcium | 8 % |
| - Alcool cétylstéarylique/alcool cétylstéarylique oxyéthyléné (33 OE) (mélange 80/20 vendu sous la dénomination Cire Lanol CTO par la société Seppic) (émulsionnant) | 4 % |
| - Oxyde de calcium anhydre (charge) | 3 % |
| - EXPANCEL 551 DE 20 | 3 % |
| - Eau | qsp100 % |

On obtient une pâte blanche lisse, au toucher doux, facilement modelable, d'application et d'élimination faciles.

Les particules ont l'avantage, en outre, d'absorber fortement l'odeur des thiols, ce qui confère à la composition une odeur beaucoup plus faible que celle des compositions dépilatoires de l'état de la technique.

On applique, notamment à la main, cette préparation sur la partie à épiler. On laisse agir pendant 10 minutes, puis on rince soigneusement à l'eau. On constate une très bonne épilation sans aucun problème d'irritation.

## Revendications

1. Composition dépilatoire, caractérisée en ce qu'elle contient, dans un milieu cosmétiquement acceptable, un agent de ramollissement des poils et un agent structurant insoluble dans ce milieu et formé de particules solides, conférant à la composition un aspect de solide déformable dans lequel le milieu est emprisonné, cet agent étant apte à s'éliminer de la peau au moyen d'un diluant.

2. Composition selon la revendication 1, caractérisée en ce que les particules ont une granulométrie de 1 µm à 300 µm.

3. Composition selon la revendication 1 ou 2, caractérisée en ce que les particules ont une granulométrie de 10 µm à 100 µm.

4. Composition selon l'une des revendications précédentes, caractérisée en ce que les particules ont une densité inférieure à 0,09.

5. Composition selon l'une quelconque des revendications précédentes, caractérisée en ce que les particules ont une densité inférieure à 0,04.

6. Composition selon l'une quelconque des revendications précédentes, caractérisée en ce que les particules sont présentes en une concentration au moins égale à la charge pigmentaire volumique critique.

7. Composition selon l'une quelconque des revendications précédentes, caractérisée en ce que les particules sont creuses.

8. Composition selon l'une quelconque des revendications précédentes, caractérisée en ce que les particules sont en matériau thermoplastique.

9. Composition selon l'une quelconque des revendications précédentes, caractérisée en ce que les particules sont réalisées en un matériau choisi parmi le verre, le Nylon, les polymères et copolymères de chlorure de vinylidène, d'acrylonitrile et/ou d'un monomère acrylique ou styrénique.

10. Composition selon l'une quelconque des revendications précédentes, caractérisée en ce que les particules sont des particules creuses de copolymère de chlorure de vinylidène, d'acrylonitrile et/ou d'un monomère acrylique ou styrénique, expansé.

11. Composition selon l'une quelconque des revendications précédentes, caractérisée en ce que l'agent de ramollissement des poils est choisi parmi les thiols.

12. Utilisation de la composition selon l'une quelconque des revendications précédentes pour l'élimination des poils.

13. Procédé d'épilation de la peau, consistant à appliquer sur la peau une composition selon l'une quelconque des revendications 1 à 11, à laisser agir, puis à rincer la peau.

14. Utilisation d'un agent structurant dans une composition dépilatoire pour lui conférer un aspect de solide déformable, cet agent étant formé de particules solides et étant apte à être éliminé au moyen d'un diluant.

15. Utilisation selon la revendication 14, caractérisée en ce que les particules ont une granulométrie allant de 1 µm à 300 µm.

16. Utilisation selon la revendication 14 ou 15, caractérisée en ce que les particules ont une granulométrie allant de 10 µm à 100 µm.

17. Utilisation selon l'une des revendications 14 à 16, caractérisée en ce que les particules ont une densité inférieure à 0,09.

18. Utilisation selon l'une des revendications 14 à 17, caractérisée en ce que les particules ont une densité inférieure à 0,04.

19. Utilisation selon l'une des revendications 14 à 18, caractérisée en ce que les particules sont présentes en une concentration au moins égale à la charge pigmentaire volumique critique.

20. Utilisation selon l'une des revendications 14 à 19, caractérisée en ce que les particules sont creuses.

21. Utilisation selon l'une des revendications 14 à 20, caractérisée en ce que les particules sont en matériau thermoplastique.

22. Utilisation selon l'une des revendications 14 à 21, caractérisée en ce que les particules sont réalisées en un matériau choisi parmi le verre, le Nylon, les polymères et copolymères de chlorure de vinylidène, d'acrylonitrile et/ou d'un monomère acrylique ou styrénique.

23. Utilisation selon l'une des revendications 14 à 22, caractérisée en ce que les particules sont des particules creuses de copolymère de chlorure de vinylidène, d'acrylonitrile et/ou d'un monomère acrylique ou styrénique, expansé.

24. Utilisation selon l'une des revendications 14 à 23, caractérisée en ce que la composition contient, en outre, au moins un agent de ramollissement des poils choisi parmi les thiols.

## Claims

1. Hair-removing composition, characterized in that it contains, in a cosmetically acceptable medium, a hair-softening agent and a structuring agent which is insoluble in this medium and formed of solid particles, which imparts a deformable solid appearance to the composition in which the medium is contained, this agent being capable of being removed from the skin using a diluent.

2. Composition according to Claim 1, characterized in that the particles have a particle size of from 1 µm to 300 µm.

3. Composition according to Claim 1 or 2, characterized in that the particles have a particle size of from 10 µm to 100 µm.

4. Composition according to one of the preceding claims, characterised in that the particles have a density of less than 0.09.

5. Composition according to any one of the preceding claims, characterized in that the particles have a density of less than 0.04.

6. Composition according to any one of the preceding claims, characterized in that the particles are at a concentration at least equal to the critical pigment charge volume.

7. Composition according to any one of the preceding claims, characterized in that the particles are hollow.

8. Composition according to any one of the preceding claims, characterized in that the particles are made of a thermoplastic material.

9. Composition according to any one of the preceding claims, characterized in that the particles are made of a material chosen from glass, nylon, polymers and copolymers of vinylidene chloride, of acrylonitrile and/or of an acrylic or styrene monomer.

10. Composition according to any one of the preceding claims, characterized in that the particles are hollow particles of copolymer of vinylidene chloride, of acrylonitrile and/or of an acrylic or styrene monomer, which is expanded.

11. Composition according to any one of the preceding claims, characterized in that the hair-softening agent is chosen from thiols.

12. Use of the composition according to any one of the preceding claims for hair removal.

13. Process for removing hair from the skin, consisting in applying a composition according to any one of Claims 1 to 11 to the skin, in allowing the composition to act and then in rinsing the skin.

14. Use of a structuring agent in a hair-removing composition in order to impart a deformable solid appearance thereto, this agent being formed of solid particles and being capable of being removed using a diluent.

15. Use according to Claim 14, characterized in that the particles have a particle size ranging from 1 µm to 300 µm.

16. Use according to Claim 14 or 15, characterized in that the particles have a particle size ranging from 10 µm to 100 µm.

17. Use according to one of Claims 14 to 16, characterized in that the particles have a density of less than 0.09.

18. Use according to one of Claims 14 to 17, characterized in that the particles have a density of less than 0.04.

19. Use according to one of Claims 14 to 18, characterized in that the particles are at a concentration at least equal to the critical pigment charge volume.

20. Use according to one of Claims 14 to 19, characterized in that the particles are hollow.

21. Use according to one of Claims 14 to 20, characterized in that the particles are made of a thermoplastic material.

22. Use according to one of Claims 14 to 21, characterized in that the particles are made of a material chosen from glass, nylon, polymers and copolymers of vinylidene chloride, of acrylonitrile and/or of an acrylic or styrene monomer.

23. Use according to one of Claims 14 to 22, characterized in that the particles are hollow particles of copolymer of vinylidene chloride, of acrylonitrile and/or of an acrylic or styrene monomer, which is expanded.

24. Use according to one of Claims 14 to 23, characterized in that the composition contains, moreover, at least one hair-softening agent chosen from thiols.

## Patentansprüche

1. Zusammensetzung zur Haarentfernung, dadurch gekennzeichnet, daß sie in einem kosmetisch akzeptablen Medium ein Mittel zum Weichmachen von Haaren und ein aus festen Partikeln gebildetes, in diesem Medium unlösliches strukturbeeinflussendes Mittel enthält, das der Zusammensetzung das Aussehen eines verformbaren Feststoffs verleiht, in dem das Medium eingeschlossen ist, wobei dieses Mittel mit einem Verdünnungsmittel von der Haut entfernt werden kann.

2. Zusammensetzung nach Anspruch 1, dadurch gekennzeichnet, daß die Partikel eine Teilchengröße von 1 bis 300 µm aufweisen.

3. Zusammensetzung nach Anspruch 1 oder 2, dadurch gekennzeichnet, daß die Partikel eine Teilchengröße von 10 bis 100 µm aufweisen.

4. Zusammensetzung nach einem der vorhergehenden Ansprüche, dadurch gekennzeichnet, daß die Partikel eine Dichte unter 0,09 aufweisen.

5. Zusammensetzung nach einem der vorhergehenden Ansprüche, dadurch gekennzeichnet, daß die Partikel eine Dichte unter 0,04 aufweisen.

6. Zusammensetzung nach einem der vorhergehenden Ansprüche, dadurch gekennzeichnet, daß die Partikel in einer Konzentration enthalten sind, die mindestens so groß ist wie die kritische spezifische Pigmentfülltstoffmenge (CPVC).

7. Zusammensetzung nach einem der vorhergehenden Ansprüche, dadurch gekennzeichnet, daß die Partikel hohl sind.

8. Zusammensetzung nach einem der vorhergehenden Ansprüche, dadurch gekennzeichnet, daß die Partikel aus einem thermoplastischen Material bestehen.

9. Zusammensetzung nach einem der vorhergehenden Ansprüche, dadurch gekennzeichnet, daß die Partikel aus einem Material hergestellt werden, das unter Glas, Nylon, Polymeren und Copolymeren aus Vinylidenchlorid, Acrylnitril und/oder einem Acryl- oder Styrolmonomer ausgewählt ist.

10. Zusammensetzung nach einem der vorhergehenden Ansprüche, dadurch gekennzeichnet, daß die Partikel Hohlpartikel aus einem expandierten Copolymer aus Vinylidenchlorid, Acrylnitril und/oder einem Acryl- oder Styrolmonomer sind.

11. Zusammensetzung nach einem der vorhergehenden Ansprüche, dadurch gekennzeichnet, daß das Mittel zum Weichmachen der Haare unter Thiolen ausgewählt ist.

12. Verwendung der Zusammensetzung nach einem der vorhergehenden Ansprüche zur Entfernung von Haaren.

13. Verfahren zum Entfernen von Haaren, das darin besteht, auf die Haut eine Zusammensetzung nach einem der Ansprüche 1 bis 11 aufzutragen, einwirken zu lassen und anschließend die Haut abzuspülen.

14. Verwendung eines strukturbeeinflussenden Mittels in einer Zusammensetzung zur Haarentfernung, um der Zusammensetzung das Aussehen eines verformbaren Feststoffs zu verleihen, wobei dieses Mittel aus festen Partikeln gebildet wird und mit einem Verdünnungsmittel entfernt werden kann.

15. Verwendung nach Anspruch 14, dadurch gekennzeichnet, daß die Partikel eine Teilchengröße im Bereich von 1 bis 300 µm aufweisen.

16. Verwendung nach Anspruch 14 oder 15, dadurch gekennzeichnet, daß die Partikel eine Teilchengröße im Bereich von 10 bis 100 µm aufweisen.

17. Verwendung nach einem der Ansprüche 14 bis 16, dadurch gekennzeichnet, daß die Partikel eine Dichte unter 0,09 aufweisen.

18. Verwendung nach einem der Ansprüche 14 bis 17, dadurch gekennzeichnet, daß die Partikel eine Dichte unter 0,04 aufweisen.

19. Verwendung nach einem der Ansprüche 14 bis 18, dadurch gekennzeichnet, daß die Partikel in einer Konzentration enthalten sind, die mindestens so groß ist wie die kritische spezifische Pigmentfüllstoffmenge (CPVC).

20. Verwendung nach einem der Ansprüche 14 bis 19, dadurch gekennzeichnet, daß die Partikel hohl sind.

21. Verwendung nach einem der Ansprüche 14 bis 20, dadurch gekennzeichnet, daß die Partikel aus einem thermoplastischen Material bestehen.

22. Verwendung nach einem der Ansprüche 14 bis 21, dadurch gekennzeichnet, daß die Partikel aus einem Material hergestellt werden, das unter Glas, Nylon, Polymeren und Copolymeren aus Vinylidenchlorid, Acrylnitril und/oder einem Acryl- oder Styrolmonomer ausgewählt wird.

23. Verwendung nach einem der Ansprüche 14 bis 20, dadurch gekennzeichnet, daß die Partikel Hohlpartikel aus einem expandierten Copolymer aus Vinylidenchlorid, Acrylnitril und/oder einem Acryl- oder Styrolmonomer sind.

24. Verwendung nach einem der Ansprüche 14 bis 23, dadurch gekennzeichnet, daß die Zusammensetzung ferner mindestens ein Mittel zum Weichmachen der Haare enthält, das unter Thiolen ausgewählt wird.
